# EUROPEAN PATENT APPLICATION

(11) **EP 2 865 396 A1**
(43) Date of publication of application: **29.04.2015**
(21) Application number: 13189646.6
(22) Date of filing: 22.10.2013
(51) Int. Cl.: A61L 27/18, A61L 27/48, A61F 2/38

(54) **Meniscus prosthesis**

(71) Applicant: Stichting Katholieke Universiteit, 6525 EZ Nijmegen (NL); DSM IP Assets B.V., 6411 TE Heerlen (NL)
(72) Inventor: Buma, Pieter, 6546 VE Nijmegen (NL); Mannel, Henrich, 12203 Berlin (DE); Nickel, Reimo, 6411 TE Heerlen (NL); Schreiber, Fabian, 52249 Eschweiler (DE); Schumann, Detlef, 6411 TE Heerlen (NL); Tienen van, Tony George, 6511 KC Nijmegen (NL)
(74) Representative: Scheltus, Irma

(57) **Abstract**

The invention is directed to a meniscus prosthesis comprising a prosthesis body, a fibrous structure that fully encloses the prosthesis body and an outer layer covering at least a part of the fibrous structure, wherein the prosthesis body, the fibrous structure and the outer layer are made of a biocompatible, non-resorbable material, and wherein the fibrous structure preferably is obtained by knitting, braiding or webbing at least one fiber.

## Description

The invention is directed to a meniscus prosthesis, a process for the production of a meniscus prosthesis and to a method for replacing at least a part of a natural meniscus by a meniscus prosthesis.

The meniscus distributes loads from the femur to the tibia plateau and by its adaptation to the contour of the joint, together with its low friction surface; it provides a smooth nearly frictionless motion of the knee joint. The highly oriented circumferential and radial collagen bundles make the matrix of the meniscus highly anisotropic. Tears (damages) can occur in the meniscus, causing pain and function loss of the knee joint. When tears occur in the meniscus generally a part of the meniscus tissue or the meniscus itself has to be removed. Removal of meniscus tissue may lead to serious osteoarthritic degeneration of the knee joint, especially when a (sub)total menisectomy was necessary. A meniscus prosthesis would postpone or even prevent other extensive and expensive knee surgeries, such as a total knee replacement.

By replacing the ectomized meniscus by an artificial implant the normal joint homeostasis would be restored, the pain could diminish, the function could be restored and further osteoarthritic degeneration could be prevented. Likely this would reduce the cost of healthcare since the number of expensive joint replacement procedures would be reduced.

In WO2011/138045 a non-resorbable meniscus prosthesis is described that is made of a biocompatible material. The non-resorbable meniscus comprises bone plugs and/or sutures for the fixation of the meniscus prosthesis in the knee joint. This meniscus prosthesis has the disadvantage that it is not strengthened, by fibers or in any other way, in the circumferential direction. This means that this meniscus implant might show some deformation when it is under pressure for a long time.

US2009/0259311 describes a prosthetic device for use as an artificial meniscus comprising a central portion and an side portion around or next to the central portion. The prosthetic device comprises at least one reinforcing fiber embedded in the side portion of the artificial meniscus. Instead of the reinforcing fiber(s) also a fiber mesh reinforcing structure can be used. The prosthetic device of US2009/0259311 has a limited stiffness and strength.

The present invention solves these disadvantages by providing a meniscus prosthesis comprising a prosthesis body, a fibrous structure that fully encloses the prosthesis body and an outer layer covering at least a part of the fibrous structure, wherein the prosthesis body, the fibrous structure and the outer layer are made of a biocompatible, non-resorbable material.

The meniscus prosthesis according to the invention has the advantage that the presence of the fibrous structure fully enclosing the prosthesis body will prevent unwanted deformation and damage of the meniscus prosthesis when the prosthesis has been implanted in a human or animal body. The reinforcement of the prosthesis body is provided over the whole of the meniscus prosthesis.

A further advantage of the prosthesis of the invention is that the outer layer provides the prosthesis with a smooth surface allowing easy motion of the knee joint after implantation and shows minimal abrasive wear..

A biocompatible material is a material that is biologically compatible by not producing a toxic, injurious, or immunological response in living tissue. A non-resorbable material is a material that shows no to very limited in-vivo degradation. In-vivo degradation is degradation of the material that occurs in the human or animal body. In-vivo degradation can for instance occur by enzymatic or hydrolytic degradation.

The meniscus prosthesis according to the invention comprises a prosthesis body. The prosthesis body is made of a biocompatible, non-resorbable material, preferably a polymeric material.

The polymeric material of the prosthesis body can, for example, be selected from the group comprising a hydrogel and/or thermoplastic material, for example polyacrylonitrile polymers and polyvinylalcohol hydrogels, elastomers, polypropylene, polyethylene, polyetheretherketones (PEEK), silicon rubbers, and polyurethanes. Combinations of these polymeric materials can also be used.

Such materials together with the design of the meniscus prosthesis body provide the required properties to the meniscus prosthesis body, e.g. high strength, high strain at break, flexibility and high wear resistance.

Preferably, the polymeric material used in the prosthesis body comprises a polyurethane and more preferably a polycarbonate urethane. Polycarbonate urethanes were the first biomedical polyurethanes promoted for their biostability. These polyurethanes include, but are not limited to the following: Bionate® a polycarbonate-urethane, PurSil® a Silicone Polyether Urethane and CarboSil ® a Silicone Polycarbonate Urethane, Elasthane® a Polyether based Polyurethane manufactured by DSM Biomedical Inc. ("DSM"); ChronoFlex® and Hydrothane, manufactured by CARDIOTECH CTE; Tecothante® (aromatic polyether-based polyurethane), Carbothane® (aliphatic polycarbonate-based polyurethane), Tecophilic®. (aliphatic polyether-based polyurethane) and Tecoplast® (aromatic polyether-based polyurethane), manufactured by THERMEDICS; Elast-Eon®, manufactured by AorTech Biomaterials and Texin®, manufactured by Bayer Corporation.

The prosthetic body preferably resembles the shape of a natural meniscus. A natural meniscus is a meniscus in a knee of a human or a mammal.

It is possible to determine the anatomical meniscus shape of the natural meniscus by performing magnetic resonance imaging (MRI) and/or computer tomography (CT) and/or X-ray scans on a meniscus in a human or a mammal.

By performing MRI and/or CT and/or X-ray scans a three dimensional (3D) image can be made of the natural meniscus of an individual which can be imitated and be resembled in a mold that is made to resemble the shape of the natural meniscus. It is also possible to determine the anatomical meniscus shape for a group of individuals by performing MRI and/or CT and/or X-ray scans on a group of individuals and to make a mold based on the average meniscus shape of the group of individuals. It is preferred to have a meniscus prosthesis that is resembling the shape of an individual's natural meniscus to provide optimal fit, load transfer and pressure distribution in all positions of the joint in an individual after implantation. This will optimally stabilize the knee joint, reduce unwanted contact stresses and will relieve the pain of an individual.

The meniscus prosthesis further comprises a fibrous structure that fully encloses the prosthesis body. The fibrous structure can be prepared using one fiber or a plurality of fibers. The fibers can for instance be knitted, braided, weaved or webbed to make the fibrous structure that encloses the prosthesis body. Preferably the fibrous structure is obtained by knitting, braiding or webbing at least one fiber. More preferably, the fibrous structure is obtained by three-dimensional (3D) braiding or knitting at least one fiber. By knitting, braiding or webbing a dense fibrous structure can be obtained that fully encloses the prosthesis body and will reinforce the whole prosthesis body. The dense fibrous structure will improve the stiffness and strength of the complete meniscus prostheses. The advantage of 3D braiding or knitting is that by using these techniques a fibrous structure can be obtained that closely follows the shape of the prosthesis body and tightly encloses the prosthesis body.

The fibrous structure closely follows the shape of the prosthesis body. It forms an envelope around the prosthesis body and completely encloses the prosthesis body.

The fiber used to prepare the prosthesis body can be any biocompatible and non-resorbable fiber. Combinations of different fibers can be used. The fiber can for example be chosen from fibers made of Ultra High Molecular Weight Polyethylene (UHMWPE), for example DSM Dyneema® Purity; polyamide, for example DuPont® Kevlar, Kevlar29, Kevlar49; polyvinylidene fluoride (PVDF);polyester, for example Ethibond Excel ®; nylon; ceramic; carbon; stainless-steel; titanium; nickel-titanium (Nitinol); and/or other suitable fibers. The fiber is preferably chosen from a UHMWPE fiber, a polyamide fiber or a polyvinylidene fluoride fiber.

The fibers may be employed in a monofilament or multifilament form as a single strand or a multiple fiber twine. When more than one fiber is used the fibers can be twisted into a yarn. The mass density of the fiber is between 500 to 1500 decitex (dtex), preferably 550 to 1000 dtex, more preferably 500 to 800 dtex. The average Elongation at Break, as determined according to ISO 2062, of the fiber preferably is between 300 and 450%, more preferably between 320 and 440%, most preferably between 340 and 420%. The Breaking Force of the fibers preferably is between 400 and 600 cN, as determined according to ISO 2062, more preferably between 420 and 570 cN, most preferably between 450 and 550 cN.

The meniscus prosthesis according to the invention also comprises an outer layer covering at least a part of the fibrous structure. Also the outer layer is made of a biocompatible, non-resorbable material, preferably a polymeric material. The outer layer can comprise the same polymeric materials as are used for the prosthesis body. For example, a hydrogel material, elastomers, polypropylene, polyethylene, polyetheretherketones (PEEK), silicon rubbers, and polyurethanes. Preferably, a polycarbonate urethane, as described above, can be used as a polymeric material in the outer layer. It is important that the outer layer provides the meniscus prosthesis with a surface that allows easy motion of the knee joint after implantation of the meniscus prosthesis. The polymeric material of the outer layer can comprise additives, end-group modifications or other surface modifications, like for example coatings, that support the easy motion of the knee joint. End-group modification of polyurethanes is for example described in US5589563, WO04044012, WO07142683 and WO08150788. The polymeric material can also be modified, for example by grafting, to provide a meniscus prosthesis with an even smoother surface. The outer layer covers at least a part of the fibrous structure. Preferably, at least the part of the meniscus prosthesis that is in contact with the femur during use is provided with the outer layer.

The fibrous structure preferably comprises, on at least one end, one or more fibers that can be used for the fixation of the prosthesis. Preferably, one or more fibers are present on two ends of the fibrous structure. These fibers for fixation of the prosthesis can be fibers that form part of the fibrous structure and are long enough to be used for fixation of the prosthesis. It is also possible that the fibers for fixation of the prosthesis are attached to the fibrous structure, during formation of the fibrous structure or after formation of the fibrous structure. Attachment to the fibrous structure can for example be performed by braiding in or knitting in the fiber or by attaching it by stitching.

The fiber material that is mentioned for employment in the fibrous structure can also be used for the fibers that are used for fixation of the prosthesis.

Fixation of the prosthesis can be performed by fixation of the prosthesis in the knee joint onto the surface of the tibia. The fibers, for example, can be used for fixation of the meniscus prosthesis to the tibia plateau. The fibers are preferably of the same material as the fibers used in the fibrous structure. More preferably the fibrous structure comprises fibers that can be used for fixation of the prosthesis on both ends of the fibrous structure.

The invention is also directed to a process for the production of the meniscus prosthesis according to the present invention, wherein the process comprises the following steps:
a) Molding the polymeric material in a mold to form the prosthesis body;
b) Forming the fibrous structure around the prosthesis body and
c) Covering at least a part of the fibrous structure with the outer layer.

Molding the polymeric material in the mold to form the prosthesis body is preferably performed by molding the polymeric material above the melting point of the polymeric material. Thereafter the polymeric material is cooled and the prosthesis body is removed from the mold. Molding can be performed e.g. by injection molding, extrusion or compression molding.

Preferably, the fibrous structure is formed around the prosthesis body by braiding, knitting or webbing. More preferably, the fibrous structure is formed by three dimensional (3D) braiding or 3D knitting. For braiding a braiding machine can be used and for knitting a knitting machine. To form the fibrous structure around the prosthesis body the prosthesis body is fixed in the braiding or knitting machine and at least one fiber is braided, knitted or webbed around the prosthesis body to form a fibrous structure fully enclosing the prosthesis body.

The outer layer for covering at least a part of the fibrous structure can, for example, be formed by dipping the prosthesis body enclosed by the fibrous structure in a solution of a polymer. Also spraying the polymer solution onto the prosthesis body that is enclosed by the fibrous structure is possible. During covering the prosthesis body enclosed with the fibrous structure by, for example, dipping or spraying of the polymer solution, the fibrous structure will not only be covered by the outer layer, but also impregnation of the fibrous structure with the polymer solution can take place. By impregnation of the fibrous structure with the outer layer the fibrous structure becomes embedded in the outer layer. The outer layer can fully or partly enclose the fibrous structure. Preferably, the fibrous structure is at least partly embedded in the outer layer.

The polymers that can be used to form the outer layer are mentioned above. The solution of the polymer can, for example, be made in suitable solvents like dimethylformamide (DMF), dimethylacetamide (DMAc) or tetrahydrofuran (THF). The concentration of the polymer in the solution preferably is 1 to 40 wt% based on the total weight of the solution, more preferably 5 to 20 wt%, most preferably 10 to 15 wt%. After the outer layer is applied to the fibrous structure the solvent is evaporated at room temperature or a higher temperature. This higher temperature is at most 100 ºC, preferably at most 75 ºC. Drying is also possible in a vacuum oven under controlled conditions.

Before molding of the polymeric material the following steps can be performed to be able to prepare a meniscus prosthesis resembling a natural meniscus:
a) Performing MRI and/or CT and/or X-ray scans on the meniscus of at least one individual;
b) Determining an anatomical meniscus shape based on the data obtained with the scans;
c) Making a mold resembling the anatomical meniscus shape.

The mold resembling the anatomical meniscus shape can, for example, be formed by the two methods described here below.;
- The mold is formed by making a body having the anatomical meniscus shape by rapid prototyping and forming the mold around the body or
- The mold is formed by 3D machining.

The invention is further directed to a method for replacing at least a part of a natural meniscus by a meniscus prosthesis according to the invention. Replacement can for instance be performed when the natural meniscus was (partly) removed in the past or when the natural meniscus is removed because it is newly damaged. Preferably, during the method at least a part of a damaged natural meniscus is replaced by a meniscus prosthesis according to the invention or a complete natural meniscus is replaced.

The invention will be further explained according to the following specific examples, without being limited thereto.

### Examples

The shape of a healthy meniscus of a group of 13 persons was determined by performing MRI scans. A computer model of the shape of an average human meniscus was made based on the collected data. An aluminum mold was prepared based on the computer model of an average human meniscus. A polymer (Bionate® 80A of DSM) was injection molded in the mold to form the prosthesis body. Around this meniscus a fibrous structure was formed by overbraiding polyvinylidene fluoride (PVDF) yarn on a Herzog braiding machine. The yarn consisted of 30 filaments. The prosthesis body was fixed between the bed plate and the take-up of the braiding machine. The prosthesis body was overbraided with 24 yarns and the braiding angle was 79º. A 0º yarn was implemented in the fibrous structure. The 0º yarn can be used as a suture for fixation of both ends of the meniscus prosthesis. The machine and take-up speed of the braiding machine was 35 rounds/min (horngear speed) and 12 picks/cm.

A solution is made comprising 15 wt% of Bionate® 80A in THF. The meniscus prosthesis, with the exception of the 0º yarn, is dipped into this solution under vacuum at room temperature for 300 sec. Thereafter the meniscus prosthesis is dried at 50 ºC for 4 hours. This process is repeated 3 times. After the last cycle the drying time was 24h at 50 ºC under vacuum.

A meniscus prosthesis was obtained having favorable mechanical properties.

## Claims

1. Meniscus prosthesis comprising a prosthesis body, a fibrous structure that fully encloses the prosthesis body and an outer layer covering at least a part of the fibrous structure, wherein the prosthesis body, the fibrous structure and the outer layer are made of a biocompatible, non-resorbable polymeric material.

2. Meniscus prosthesis according to claim 1, wherein the fibrous structure is obtained by knitting, braiding or webbing at least one fiber.

3. Meniscus prosthesis according to claim 1 or 2, wherein the fibrous structure is made from a UHMWPE fiber, a polyamide fiber or a polyvinylidene fluoride fiber.

4. Meniscus prosthesis according to claim 3, wherein the fibrous structure has a mass density between 500 to 1500 dtex.

5. Meniscus prosthesis according to any one of claims 1-4, wherein the prosthesis body comprises a polyurethane.

6. Meniscus prosthesis according to claim 5, wherein the polyurethane is a polycarbonate-urethane.

7. Meniscus prosthesis according to any one of claims 1-6, wherein the fibrous structure is at least partly embedded in the outer layer.

8. Meniscus prosthesis according to any one of claims 1-7, wherein at least one end of the fibrous structure comprises one or more fibers that can be used for the fixation of the prosthesis.

9. Process for the production of the meniscus prosthesis according to any one of claims 1-8, wherein the process comprises the following steps:
a) Molding the polymeric material in a mold to form the prosthesis body;
b) Forming the fibrous structure around the prosthesis body and
c) Covering at least a part of the fibrous structure with the outer layer.

10. Process according to claim 9, wherein the fibrous structure is formed around the prosthesis body by braiding, knitting or webbing.

11. Process according to claim 9 or 10, wherein the fibrous structure is formed by 3D braiding or 3D knitting.

12. Process according to any one of claims 9-11, wherein, the mold used in step a) is made by a process comprising the following steps:
a. Performing MRI and/or CT and/or X-ray scans on the meniscus of at least one individual;
b. Determining an anatomical meniscus shape based on the data obtained with the scans and
c. Making a mold resembling the anatomical meniscus shape.

13. Process according to claim 12, wherein the mold resembling the anatomical meniscus shape is formed by making a body having the anatomical meniscus shape by rapid prototyping and forming the mold around the body; or by 3D machining.

14. Method for replacing at least a part of a natural meniscus by the meniscus prosthesis according to any one of claims 1-8.

15. Method according to claim 14, wherein at least a part of a damaged natural meniscus is replaced or wherein a complete natural meniscus is replaced.
